# EUROPEAN PATENT SPECIFICATION

(11) **EP 0 138 216 B1**
(45) Date of publication and mention of the grant of the patent: **07.01.1993**
(21) Application number: 84112312.8
(22) Date of filing: 12.10.1984
(51) Int. Cl.: A61K 9/22, A61K 37/66, A61K 47/00

(54) **Sustained-release IFN preparation for parenteral administration**
IFN-Präparat mit verzögerter Abgabe für parentale Verabreichung
Préparation IFN à libération prolongée pour administration par voie parentérale

(30) Priority: 14.10.1983 JP 193064/83; 20.10.1983 JP 197181/83; 01.11.1983 JP 206226/83
(43) Date of publication of application: 24.04.1985
(73) Proprietor: SUMITOMO PHARMACEUTICALS COMPANY, LIMITED, Osaka-shi, Osaka-fu 541 (JP)
(72) Inventor: Yamahira, Yoshiya, Kobe-shi Hyofo-ken (JP); Fujioka, Keiji, Ibaraki-shi Osaka-fu (JP); Sato, Shigeji, Ibaraki-shi Osaka-fu (JP); Yoshida, Noboru, Osaka-shi Osaka-fu (JP)
(74) Representative: VOSSIUS & PARTNER

(56) References cited:
- EP-A- 0 094 157
- EP-A- 0 098 110
- EP-A- 0 134 289
- WO-A-83/01198
- DE-A- 3 104 815
- GB-A- 2 042 888
- GB-A- 2 067 072
- US-A- 4 181 731

## Description

The present invention relates to a sustained-release preparation of interferon for parenteral administration.

It is known that interferons are produced in the cells of animals and humans by irritation with virus or any other substance. They are very useful as an agent for inhibiting growth of viruses and as an anti-tumor agent. Recently, clinical tests of interferons have been done for investigating the activities against various virus diseases and tumors. It has been expected that the activities are promoted by sustaining the level of the medicament in blood or in lesional regions. However, such a sustained-release preparation suitable for parenteral administration has not been found.

It is very difficult to sustain the release of interferon by known techniques for release-sustaining, because interferon is very unstable not only within the body but also in the preparation. Hence the activity is largely and rapidly decreased by the conventional release-sustaining techniques such as heat treatment or irradiation, or by chemical treatments with organic solvents or aldehydes, and further, the conventional interferon preparations contain a very small amount of interferon which is water-soluble and hence interferon is easily and rapidly released when administered. Besides, the interferon preparation is a parenteral preparation such as an injection preparation, and hence, there is a problem of accumulation of the carrier within a body when the preparation is administered for a long period of time. From this viewpoint, non-biodegradable carried such as silicone which is recently used in some medical sections can not be employed.

DE-A-31 04 815 describes a process for preparing sustained release compositions wherein the active ingredient is dispersed in, fixed to and encapsulated by a protein. For said process it is essential to denaturize the protein by e.g. heat treatment or freezing (and thawing) in order to achieve the desired sustained release. On the other hand, the preparation of the present invention is optionally lyophilized. It is well known in the chemical and biological field that lyophilization, which is different to simply freezing, is rather used for drying unstable material like IFN without denaturizing it.

US-A-4 181 731 describes a composition containing 4-carbamoyl-5-hydroxyimidazole as an active ingredient and a pharmaceutically acceptable carrier. The carrier may be gelatin, a polysaccharide or a synthetic high molecular compound. This reference does not teach or suggest to prepare a sustained release preparation of IFN.

WO-A-8310198 discloses a preparation which is clearly distinguished form the sustained release preparation of the present invention in that it is an aqueous gel product and hence it is difficult to keep the water-soluble IFN stable for a long period of time.

In GB-A-2042 888 cellulose ether is used as the carrier and the drugs used therein are all low molecular weight compounds and powdery compounds in contrast to IFN.

Thus, the technical problem underlying the invention is to provide an improved sustained-release preparation of interferon for parenteral administration which can gradually release the active ingredient and can maintain the desired level of the active ingredient in blood or in the lesional region for a long period of time.

The sustained-release preparation of the present invention comprises interferon as an active ingredient in admixture with a pharmaceutically acceptable biodegradable protein as carrier, said preparation being in the form of powder particles or in the form of a shaped preparation with the proviso that the form is neither needle-like nor bar-like.

The biodegradable carrier used in the present invention means a carrier which can easily be absorbed or be subjected to enzymatic degradation in the body and can be implanted into the body. Suitable examples of the biodegradable proteins are collagen and gelatin.

These substances can be used alone or in combination; in view of safety and easy handling, collagen or gelatin or a mixture thereof are used. Collagen is a protein which is a main protein of connective tissue of animals and has less antigenicity, and hence, has widely been used as a safe operation yarn for various medical operations. The collagen may be an atelocollagen having far less antigenicity which is obtained by removing the telopeptide region by treating collagen with an enzyme (e.g. pepsin) in order to make it safer. Gelatin is a protein derived from collagen. Gelatin is a high molecular weight amphoteric electrolyte which has less antigenicity and properties of convertibility between sol and gel forms and is cheap in cost, and hence it has already been confirmed as a safe substance for medical use.

The active ingredient used in the present invention includes an interferon, which may be used alone or in combination with an interferon activator or together with one or more other medical compounds which can show additional or synergistic activities with interferon. The interferon includes α-, β-, and γ-interferons, and a mixture thereof.

The ratio of the carrier and the medicament is not critical but, for example, interferon is preferably incorporated in an amount of 10³ to 10⁸ IU per 1 mg of carrier.

The sustained-release preparation of the present invention can be prepared by the following method.

An aqueous solution of interferon is mixed with the biodegradable protein or an aqueous solution thereof. The mixture is homogeneously mixed by stirring while preventing the occurrence of foam as much as possible. The resulting mixture is optionally concentrated at a low temperature and further optionally spray-dried or lyophilized. In the preparation, there may optionally be incorporated conventional pharmaceutically acceptable additives such as stabilizers, preservatives, local anesthetic agents, and some agents for aiding formability into special shapes of preparations or for release-sustaining of the active ingredient. These additives are not specified. Suitable examples of the agents for aiding formability are methylcellulose, ethylcellulose, hydroxypropylcellulose, polyvinylpyrrolidone, glycolic acid-lactic acid copolymer, polyethylene glycol, propylene glycol or ethyl alcohol. Suitable examples of the agents for aiding release-sustaining of the active ingredient are cellulose acetate phthalate, ethylcellulose, methylcellulose, hydroxypropylmethylcellulose, calcium phosphate, lactic acid-glycolic acid copolymer, corn starch, rice starch, potato starch, cellulose or alginates.

The preparation thus obtained may be optionally processed so as to make it fit to the desired use. For example, the preparation is pulverized under cooling with dry ice or liquid nitrogen. The powder having a particle size suitable for injection may be suspended in a viscous solvent suitable for injection to give a sustained-release suspension for injection. Alternatively, the pulverized preparation and the viscous solvent may be packed in the form of a kit, and both are mixed to prepare a suspension for injection when used. Suitable examples of viscous solvents for injection are vegetable oils (e.g. peanut oil, cotton seed oil, sesame oil, castor oil, olive oil, corn oil, iodinated poppy seed oil fatty acids ethyl esters), polyethylene glycol, propylene glycol, silicone oil or medium-chain fatty acids triglycerides.

The preparation of the present invention contains the active ingredient in an amount in which the active ingredient is usually used. For example, interferon is usually contained in an amount of 10⁴ to 10⁹ IU, preferably 10⁵ to 5 x 10⁸ IU, per dosage unit.

Besides, the particles of the preparation as prepared by pulverization as mentioned above or by any other conventional pulverization methods may be compressed together with other additives if needed to form some specific shapes, such as needle-like or fine bar-like shaped preparations (diameter: about 0.5 mm - 1.5 mm, length: about 5 mm - 15 mm), which can be inserted into the body with a forceps needle for fiberscope, an indwelling needle, or other appropriate administration devices. Alternatively, the powdery preparation is previously entered into a mold, followed by concentrating at a low temperature or by lyophilizing to compress and form into a needle-like or a fine bar-like shaped preparation.

Moreover, the preparations of the present invention may be formed into specific shaped preparations such as pellets, spherical, granular or powdery shaped preparations, which can be implanted into the body or the lesional region during operation. The shapes to be formed may vary depending on the desired use and the desired degree of sustaining of the activity of the medicament. Generally speaking, the larger the shape of the preparation is, the longer is the time for sustaining of activity.

All steps for preparing the desired sustained-release preparations, carried out under sterile condition because the preparations are used as an injection or for implanting into the body.

The present invention is illustrated by the following Experiment and Examples, but should not be construed to be limited thereto.

### Experiment

As test samples an oily suspension of the interferon-collagen preparation prepared in Example 1 disclosed hereinafter (Sample I), a needle-shaped preparation of interferon-collagen prepared in Example 2 disclosed hereinafter (Sample J) and a reference (an aqueous injection preparation of α-interferon from Namalwa cells) were used. The test samples were each administered intramuscularly to rabbits,and the change of level in blood of the active ingredient with lapse of time was measured by RIA (radioimmunoassay) method. Two rabbits were used for each sample. The test samples were each administered in a dose of 10⁶ U/kg. The blood levels were measured in average in two rabbits.

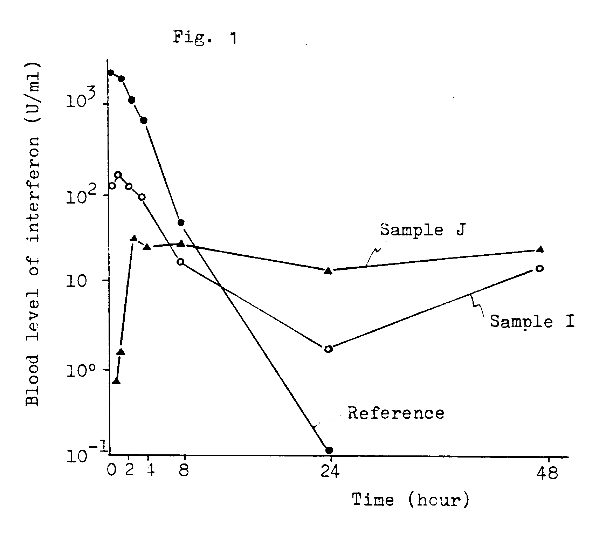

The results are shown in the accompanying Fig. 1. In Fig. 1, ○ is the graph of Sample I, ▲ is that of Sample J and ● is that of reference (α-interferon aqueous injection). As is clear from the figure, the Samples I and J showed release-sustaining, and even after 48 hours, the blood level of several tens unit/ml was maintained. The needle preparation showed particularly excellent release-sustaining.

Thus, it is also suggested by the test of in vivo using rabbits that the preparation of the present invention is useful clinically.

### Example 1

An aqueous solution of α-interferon (titer: 4.9 MU/ml) (100 ml) and 2 % atelocollagen (50 g) are homogeneously mixed with stirring with preventing occurrence of foam as small as possible. The mixture is lyophilized and pulverized at a low temperature using liquid nitrogen. The pulverized product thus obtained is suspended in sesame oil to give an oily suspension type, sustained-release preparation (Sample I) wherein interferon is contained in an amount of 4 MU per 1 vial.

### Example 2

The pulverized product in the same manner as described in Example 1 is formed under compression to give a needle-shaped sustained-release preparation (Sample J) wherein interferon is contained in an amount of 10 MU per 1 needle.

### Example 3

The pulverized product in the same manner as described in Example 1 is suspended in castor oil to give an oily suspension type, sustained-release preparation wherein interferon is contained in an amount of 4 MU per 1 vial.

### Example 4

The pulverized product in the same manner as described in Example 1 is formed into a pellet shape under compression to give a pellet-shaped sustained-release preparation wherein interferon is contained in an amount of 10 MU per 1 pellet.

### Example 5

An aqueous solution of α-interferon (titer, 4.9 MU/ml) (100 ml), 2 % atelocollagen (50 g), human serum albumin (150 mg) and thimerosal (120 µg) are homogeneously mixed with preventing occurrence of foam as small as possible. The mixture is lyophilized and pulverized at a low temperature using liquid nitrogen. The pulverized product thus obtained is suspended in sesame oil to give an oily suspension type, sustained-release preparation wherein interferon is contained in an amount of 4 MU per 1 vial.

### Example 6

An aqueous solution of α-interferon (titer, 4.9 MU/ml) (100 ml) and 2 % collagen (50 g) are homogeneously mixed with preventing occurrence of foam as small as possible. The mixture is lyophilized and pulverized at a low temperature using liquid nitrogen. The pulverized product thus obtained is suspended in sesame oil to give an oily suspension type, sustained-release preparation wherein interferon is contained in an amount of 4 MU per 1 vial.

### Example 7

An aqueous solution of α-interferon (titer, 4.9 MU/ml) (100 ml) and atelocollagen powder (1 g) are mixed and thereto is added 0.1 N hydrochloric acid, and the resulting solution is entered into a mold and lyophilized. The lyophilized product is formed under compression to give a needle-shaped sustained-release preparation wherein interferon is contained in an amount of 10 MU per 1 needle.

### Example 8

An aqueous solution of α-interferon (titer, 4.9 MU/ml) (100 ml) and gelatin (1 g) are homogeneously mixed at 60°C with preventing occurrence of foam as small as possible. The mixture is lyophilized and pulverized at a low temperature using liquid nitrogen. The pulverized product thus obtained is suspended in sesame oil to give an oily suspension type, sustained-release preparation wherein interferon is contained in an amount of 4 MU per 1 vial.

### Example 9

The pulverized product prepared in the same manner as described in Example 1 is suspended in polyethylene glycol to give a suspension type, sustained-release preparation wherein interferon is contained in an amount of 4 MU per 1 vial.

### Example 10

To the pulverized product prepared in the same manner as described in Example 1 is added methylcellulose (25 % by weight based on the weight of the pulverized product), and thereto is added distilled water for injection so that the solid content becomes 20 % by weight. The mixture is kneaded. The kneaded product is entered in a mold, lyophilized, and then, compressed to give a needle-shaped sustained-release preparation wherein interferon is contained in an amount of 10 MU per 1 needle.

### Example 11

An aqueous solution of α-interferon (titer, 4.9 MU/ml) (100 ml), 2 % atelocollagen (50 g) and tespamin (triethylenethiophosphoramide, which is known as an antineoplastic) (98 mg) are homogeneously mixed with preventing occurrence of foam as small as possible. The mixture is lyophilized and pulverized at a low temperature using liquid nitrogen. The pulverized product thus obtained is subjected to compression molding to give a needle-shaped sustained-release preparation wherein interferon and tespamin are contained in an amount of 10 MU and about 2 mg per 1 needle, respectively.

### Example 12

The pulverized product prepared in the same manner as described in Example 1 is suspended in iodinated poppy seed oil fatty acids ethyl esters (sold by Libiodol Ultrafluid - Kodama Shoji) to give an oily suspension type, sustained-release preparation wherein interferon is contained in an amount of 4 MU per 1 vial.

## Claims

1. A sustained-release preparation for parenteral administration, which comprises interferon as an active ingredient in admixture with a pharmaceutically acceptable biodegradable protein as carrier, said preparation being in the form of powder particles or in the form of a shaped preparation, with the proviso that the form is neither needle-like nor bar-like.

2. The preparation according to claim 1, wherein the biodegradable carrier is collagen, gelatin, or mixtures thereof.

3. The preparation according to claim 1, wherein the biodegradable carrier is atelocollagen.

4. The preparation according to any one of claims 1, wherein the interferon is a α-interferon.

5. The preparation according to any one of claims 1 to 4, wherein the interferon is contained in an amount of 10⁴ to 10⁹ IU per dosage unit.

6. The preparation according to any one of claims 1 to 5, which is suitable for implanting into a body.

7. A method for preparing the sustained-release preparation according to claim 1 which comprises mixing an aqueous solution of interferon and the pharmaceutically acceptable biodegradable protein, and concentrating, spray-drying or lyophilizing the mixture.

8. The method according to claim 7, wherein the concentrated, spray-dried or lyophilized mixture is pulverized to produce the powder particles.

9. The method according to claim 7, wherein the preparation is shaped by compressing the concentrated, spray-dried or lyophilized mixture or by allowing the said mixture to stand in a mold until formed.

## Patentansprüche

1. Langzeitwirkendes Mittel zur parenteralen Verabreichung, welches Interferon als Wirkstoff mit einer Beimischung eines pharmazeutisch verträglichen, biologisch abbaubaren Proteins als Träger umfaßt, wobei das Mittel in Form von Pulverteilchen oder als Formkörper vorliegt, mit der Maßgabe, daß er weder nadel- noch stäbchenrörmig ist.

2. Mittel nach Anspruch 1, wobei der biologisch abbaubare Träger Kollagen, Gelatine oder ein Gemisch davon ist.

3. Mittel nach Anspruch 1, wobei der biologisch abbaubare Träger Atelokollagen ist.

4. Mittel nach einem der Ansprüche 1 bis 3, wobei das Interferon α-Interferon ist.

5. Mittel nach einem der Ansprüche 1 bis 4, wobei das Interferon in einer Menge von 10⁴ bis 10⁹ I.E. pro Dosiseinheit enthalten ist.

6. Mittel nach einem der Ansprüche 1 bis 5, das zum Implantieren in einen Körper geeignet ist.

7. Verfahren zur Herstellung des langzeitwirkenden Mittels nach Anspruch 1, umfassend das Vermischen einer wäßrigen Lösung des Interferons und des pharmazeutisch verträglichen biologisch abbaubaren Proteins und Aufkonzentrieren, Sprüh- oder Gefriertrocknen des Gemisches.

8. Verfahren nach Anspruch 7, wobei das aufkonzentrierte, sprüh- oder gefriergetrocknete Gemisch zur Herstellung von Pulverteilchen pulverisiert wird.

9. Verfahren nach Anspruch 7, wobei das Mittel durch Pressen des aufkonzentrierten, sprüh- oder gefriergetrockneten Gemisches geformt oder das Gemisch in einer Form bis zur Ausbildung einer Gestalt stehengelassen wird.

## Revendications

1. Préparation à libération prolongée destinée à l'administration parentérale, comprenant de l'interféron comme principe actif mélangé à un véhicule constitué par une protéine biodégradable pharmaceutiquement acceptable, ladite préparation se présentant sous la forme de particules de poudre ou sous la forme d'une préparation façonnée, à la condition que cette forme ne soit ni aciculaire ni de type barres.

2. Préparation selon la revendication 1, dans laquelle le véhicule biodégradable est du collagène, de la gélatine ou leurs mélanges.

3. Préparation selon la revendication 2, dans laquelle le véhicule biodégradable est de l'atélocollagène.

4. Préparation selon l'une quelconque des revendications 1 à 3, dans laquelle l'interféron est l'interféron α.

5. Préparation selon l'une quelconque des revendications 1 à 4, dans laquelle l'interféron est contenu en une quantité de 10⁴ à 10⁹ UI par unité de prise.

6. Préparation selon l'une quelconque des revendications 1 à 5, convenant à l'implantation dans un organisme.

7. Procédé de préparation de la préparation à libération prolongée selon la revendication 1, comprenant le mélange d'une solution aqueuse d'interféron et de la protéine biodégradable pharmaceutiquement acceptable et la concentration, le séchage par pulvérisation ou la lyophilisation du mélange.

8. Procédé selon la revendication 7, dans lequel on pulvérise le mélange concentré, séché par pulvérisation ou lyophilisé pour produire des particules de poudre.

9. Procédé selon la revendication 7, dans lequel on façonne la préparation en comprimant le mélange concentré, séché par pulvérisation ou lyophilisé ou en laissant reposer ledit mélange dans un moule jusqu'à sa formation.
